(19) Europäisches Patentamt
European Patent Office
Office européen des brevets

(11) Veröffentlichungsnummer : **0 472 123 B1**

(12) **EUROPÄISCHE PATENTSCHRIFT**

(45) Veröffentlichungstag der Patentschrift :
**04.05.94 Patentblatt 94/18**

(51) Int. Cl.[5] : **C07C 49/643,** C07C 49/453, C07C 45/57, A61K 7/46

(21) Anmeldenummer : **91113762.8**

(22) Anmeldetag : **16.08.91**

# (54) Propellane.

(30) Priorität : **24.08.90 CH 2756/90**

(43) Veröffentlichungstag der Anmeldung :
**26.02.92 Patentblatt 92/09**

(45) Bekanntmachung des Hinweises auf die Patenterteilung :
**04.05.94 Patentblatt 94/18**

(84) Benannte Vertragsstaaten :
**CH DE ES FR GB IT LI NL**

(56) Entgegenhaltungen :
**EP-A- 0 315 895**
**DE-B- 2 461 593**
**FR-A- 2 523 123**
**GB-A- 2 065 108**
**US-A- 4 124 642**

(73) Patentinhaber : **GIVAUDAN-ROURE (INTERNATIONAL) S.A.**
**CH-1214 Vernier, Genève (CH)**

(72) Erfinder : **Fráter, Georg**
**Turikumstrasse 5**
**CH-8610 Uster (CH)**
Erfinder : **Helmlinger, Daniel**
**Tichelrütistrasse 18**
**CH-8044 Gockhausen (CH)**

(74) Vertreter : **Urech, Peter, Dr. et al**
**Grenzacherstrasse 124 Postfach 3255**
**CH-4002 Basel (CH)**

EP 0 472 123 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Riechstoffe, und zwar Propellane.

Die Erfindung betrifft im besonderen die Verbindungen der Formel

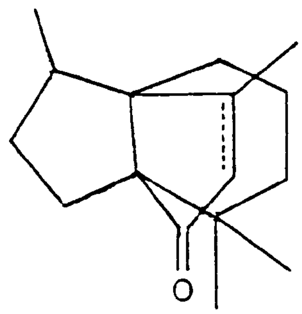

I

worin die gestrichelte Linie eine fakultative Bindung bedeutet, als Racemate oder einzelne optische Antipoden:

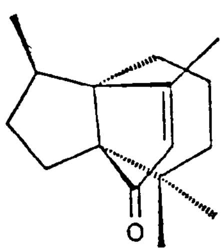

I'

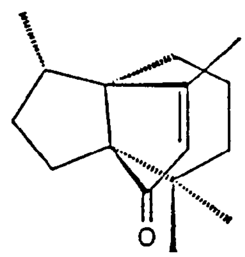

I"

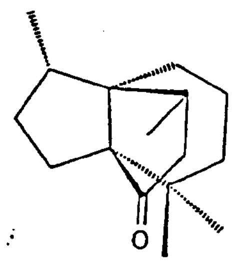

I"'

Die Verbindungen I stellen Ambrakörper dar und können demgemäss als Riechstoffe Verwendung finden.

Die Erfindung betrifft demgemäss auch die Verwendung von I als Riechstoffe.

Die Verbindungen I werden dadurch erhalten, dass man Sclareolid II bei erhöhter Temperatur in saurem Medium unter Wasserabspaltung umlagert, und die im Reaktionsprodukt vorhandene C-C-Doppelbindung gegebenenfalls reduziert.

Der Begriff "Sclareolid II" soll die folgenden Verbindungen umfassen:

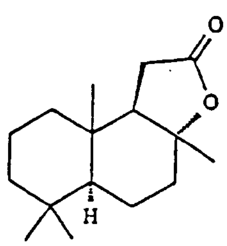

Sclareolid
II'

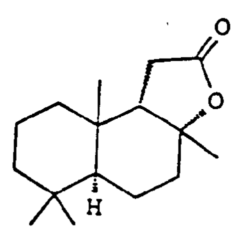

Episclareolid
II"

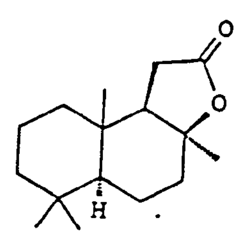

Isosclareolid
II"'

Bezüglich der Verfahrensparameter gilt folgendes:

Umlagerung unter Wasserabspaltung

| | |
|---|---|
| Temperatur: | erhöht, z.B. 70-120°C, insbesondere Temperaturen um 100°C |
| Aufarbeitung: | Zugabe von Wasser, Extraktion mit organischem Lösungsmittel |
| Lösungsmittel: | das im Ueberschuss eingesetzte phosphorhaltige Umlagerungsmittel (z.B. Polyphosphorsäure, $P_2O_5$/Methansulfonsäure) erfüllt diese Funktion |

Reduktion

| | |
|---|---|
| Temperatur: | erhöht, z.B. ca. 50-100°C, insbesondere ca. 80°C bis ca. 100°C |
| Lösungsmittel: | z.B. Toluol |
| Aufarbeitung: | Alkal. Behandlung in Anwesenheit eines organischen Lösungsmittels, Waschen, z.B. Rühren mit 2N NaOH und Aether, wässrige Phase mit 2N HCl und gesättigter Kochsalzlösung waschen |

Die Erfindung betrifft auch die Verwendung der Verbindungen I als Ambrariechstoffe.

Die Verbindungen I zeichnen sich durch kräftige, diffusive und sehr natürliche holzige, cedrige, ambrige Noten mit fruchtigen Geruchsaspekten aus. Die interessanteste Verbindung ist I''' (ausgeprägteste Ambranote), gefolgt von I' und I''.

Die Verbindungen der Formel I eignen sich aufgrund ihrer natürlichen Geruchsnoten insbesondere zur Modifizierung von bekannten Kompositionen.

Die Verbindungen I verbinden sich mit zahlreichen bekannten Riechstoffingredientien natürlichen oder synthetischen Ursprungs, wobei die Palette der natürlichen Rohstoffe sowohl leicht- als auch mittel- und schwer-flüchtige Komponenten, und diejenige der Synthetika Vertreter aus praktisch allen Stoffklassen umfassen kann, wie dies aus der folgenden Zusammenstellung ersichtlich ist:

- Naturprodukte, wie Baummoos-Absolue, Basilikumöl, Agrumenöle (wie Bergamotteöl, Mandarinenöl, etc.), Mastix-Absolue, Myrtenöl, Palmarosaöl, Patchouliöl, Petitgrainöl, Paraguay, Wermutöl,
- Alkohole, wie Farnesol, Geraniol, Linalool, Nerol, Phenyläthylalkohol, Rhodinol, Zimtalkohol,
- Aldehyde, wie Citral, Helional®, α-Hexylzimtaldehyd, Hydroxycitronellal, Lilial® (p-tert.Butyl-α-methyldihydrozimtaldehyd), Methylnonylacetaldehyd,
- Ketone, wie Allyljonon, α-Jonon, β-Jonon, Isoraldein (Isomethyl-a-ionon), Methyljonon,
- Ester, wie Allyl-phenoxyacetat, Benzyl-salicylat, Cinnamylpropionat, Citronellyl-acetat, Citronellyl-äthoxalat (Citronellyl. O - CO -CO . $OC_2H_5$), Decyl-acetat, Dimethylbenzylcarbinyl-acetat, Dimethylbenzylcarbinyl-butyrat, Aethyl-acetoacetat, Aethyl-acetylacetat, Hexenyl-isobutyrat, Linalylacetat, Methyl-dihydrojasmonat, Styrallylacetat, Vetiverylacetat,
- Lactone, wie γ-Undecalacton,
- verschiedene, in der Parfümerie oft benützte Komponenten, wie Ketonmoschus, Indol, p-Menthan-8-thiol-3-on, Methyleugenol.

Bemerkenswert ist ferner die Art und Weise, wie die Verbindungen I die Geruchsnoten bekannter Kompositionen abrunden und harmonisieren, ohne aber in unangenehmer Weise zu dominieren. So unterstreichen sie in Parfümbasen, z.B. mit Chypre, Tabak, Ambra, Leder, orientalischen und würzigem Charakter, die Ambraholz- und Leder-Note. Sie verleihen der Komposition zudem Fülle und Wärme.

Die Verbindungen der Formel I (bzw. deren Gemische) lassen sich in weiten Grenzen einsetzen, die beispielsweise von 0,1 (Detergentien) - 5% (alkoholische Lösungen) in Kompositionen reichen können, ohne dass diese Werte jedoch Grenzwerte darstellen sollen, da der erfahrene Parfümeur auch mit noch geringeren Konzentrationen Effekte erzielen oder aber mit noch höheren Dosierungen neuartige Komplexe aufbauen kann. Die bevorzugten Konzentrationen bewegen sich zwischen ca. 0,2 und 2%. Die mit I hergestellten Kompositionen lassen sich für alle Arten von parfümierten Verbrauchsgütern einsetzen (Eaux de Cologne, Eaux de Toilette, Extraits, Lotionen, Crèmes, Shampoos, Seifen, Salben, Puder, Zahnpasten, Mundwässer, Desodorantien, Detergentien, Tabak, etc.).

Die Verbindungen I können demgemäss bei der Herstellung von Kompositionen und - wie obige Zusammenstellung zeigt - unter Verwendung einer breiten Palette bekannter Riechstoffe bzw. Riechstoffgemische, verwendet werden. Bei der Herstellung solcher Kompositionen können die oben aufgeführten bekannten Riechstoffe nach (dem Parfümeur bekannter) Art und Weise verwendet werden, wie z.B. aus W.A. Poucher, Perfumes, Cosmetics and Soaps 2,7. Auflage, Chapman und Hall, London, 1974 hervorgehend.

In den folgenden Beispielen bedeuten:

| | |
|---|---|
| (-)I' | 1S,6R,10R-5,5,9,10-Tetramethyl-tricyclo-[4,3,3,$0^{1,6}$]dodec-8-en-7-on |
| (-)I'' | 1S,6R,10S-5,5,9,10-Tetramethyl-tricyclo-[4,3,3,$0^{1,6}$]dodec-8-en-7-on |
| (-)VII | 3,3a,4,5,5a,6,7,8,8,9,9a-Decahydro-6,6,9a-trimethyl[3aS-(3aa,5aβ,9aa)]-2H-benz[e]inden-2-on |

| | |
|---|---|
| (-)VIII | 1,4,5,5a,6,7,8,9,9a,9b-Decahydro-6,6,9a-trimethyl-[5aS-(5aβ,9aa,9bβ)]-2H-benz[e]inden-2-on |
| (-)I''' | 1S,6R,9S,10S-5,5,9,10-Tetramethyl-tricyclo-[4,3,3,$0^{1,6}$]dodecan-7-on |
| X | 2aR,3R,5aR,2a,3,6,6-Tetramethyl-2a,3,4,5,5a,6,7,8-octahydro-1(2H)-acenaphthylenon |
| XI | 1S,6R,10S-9-Methylen-5,5,10-trimethyl-tricyclo-[4,3,3,$0^{1,6}$]dodec-7-en |

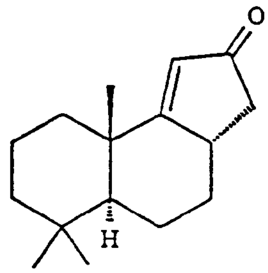

VII

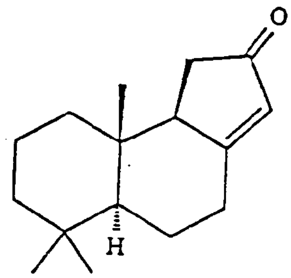

VIII

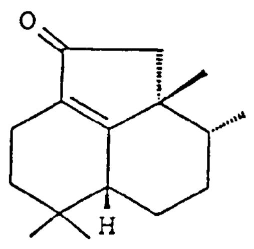

X

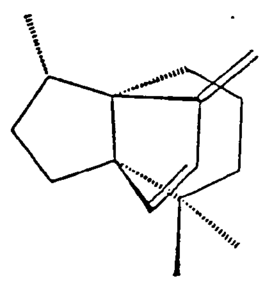

XI

Beispiel 1

150 g Polyphosphorsäure werden vorgelegt und auf 100°C erhitzt. 40 g (+)-Sclareolid II' (Decahydro-3a,6,6,9a-tetramethyl - [3aR(3aα,5aβ,9aa,9bβ)]naphto[2,1-b]furan-2(1H)-on) werden nun rasch zugegeben und das Gemisch während 1 Stunde bei 100°C gerührt. Dann wird auf 0°C abgekühlt, mit 100 ml Wasser und 50 ml Hexan 1 1/4 Stunden gerührt und mit Hexan extrahiert. Die organische Phase wird mit gesättigter Bicarbonatlösung und mit gesättigter Kochsalzlösung gewaschen, getrocknet und eingedampft. Man erhält auf diese Weise 30,8 g Rohprodukt. Dieses Produkt enthält (GC) 15% (-)I', 44% (-)I", 4,5% (-)VII, 25% (-)VIII. Eine Destillation liefert 16,6 (45%) eines Gemisches von (-)I' und (-)I" im Verhältnis 1/3,3 (Sdp. 81-89/1,47.$10^{-3}$ Pa (1,1·$10^{-5}$ Torr)) und 8,2 g (22%) eines Gemisches von (-)VII und (-)VIII im Verhältnis 1/5,4.

Die einzelnen Verbindungen (-)I', (-)I", (-)VII und (-)VIII können durch Chromatographie (Kieselgel 60, 230-400 mesh, Elution mit 20% Aether in Hexan) gereinigt werden.

Spektrale Daten und geruchliche Eigenschaften:

Verbindung (-)I'

IR: (liq) 1690, 1615 $cm^{-1}$
UV ($CH_2Cl_2$)λ max: 235, ε = 9913.
H-NMR (200 $MH_3$, $CDCl_3$): 5,97 (m,J=1,2,H-C(8)); 2,07 (d,J=1,2,$CH_3$-(C(9)); 1,03 (s,$CH_3$-C(5)); 0,95 (d,J=7,$CH_3$-C(10)); 0,79 (s,$CH_3$-C(5); $^{13}C$ NMR ($CDCl_3$): 214,1 (s,C(7)); 181,2 (s,C(9)); 132,6 (d,C(8)); 65,3 (s,C(6)); 57,6 (s,C(1)); 43,1 (d,C(10)); 34,9 (s,C(5)); 33 (t,C(4)); 31,2 (t,C(11)); 30,9 (t,C(12)); 28 (q,C(13)); 27,4 (q,C(14)); 26,3 (t,C(2)); 17,1 (q,C(15)); 16,5 (t,C(3)); 16,4 (q,C(16));
MS: 232 (40,$M^+$), 217(12), 204(2), 189(6), 175(12), 161(14), 150(100), 135(11), 122(34), 105(14), 91(15), 77(11), 69(8), 55(9), 41(17), 28(33).

$[\alpha]_D^{20}$ = -178,68° (c = 1,133, $CHCl_3$).
Holzig, cedrig, fruchtig, ambrig.

Verbindung (-)I"

IR (liq): 1690, 1620 $cm^{-1}$
UV (EtOH) λ max: 235, ε = 8754
H-NMR (200 $MH_3$, $CDCl_3$): 5,76 (m,J=1,3,H-C(8)); 2,05 (d,J=1,3,$CH_3$-C(9)); 1,05 (s,$CH_3$-C(5)eq); 1,00 (d,J=7,$CH_3$-C(10)); 0,84 (s,$CH_3$-C(5)ax); $^{13}$C NMR ($CDCl_3$): 213,8 (s,C(7)); 182,9 (s,C(9)); 129,1 (d,C(8)); 65,5 (s,C(6)); 58,9 (s,C(1)), 44,1 (d,C(10)); 34,8 (s,C(5)); 33,8 (t,C(4)); 31,8 (t,C(11)); 28,4 (t,C(12)); 26,9 (q,C(14)); 26,3 (q,C(13)); 22,9 (t,C(2)); 17,9 (t,C(3)); 15,8 (q,C(16)); 15,4 (q,C(15));
MS: 232 (34 $M^+$), 217(11), 204(6), 189(6), 175(16), 161(15), 150(100), 135(17), 122(37), 105(15), 91(16), 77(10), 69(10), 55(11), 41(21), 28(52).
$[\alpha]_D^{20}$ = -145,58° ($CHCl_3$, c = 1,047).
Geruch von (-)I": holzig, cedrig, fruchtig, ambrig.

Verbindung (-)VII

IR ($CHCl_3$): 1680, 1600 $cm^{-1}$
UV (EtOH) λ max: 232, ε = 11851
$^1$H-NMR ($CDCl_3$): 5,695-5,68 (m,H-C(1)); 2,96-2,88 (m,Ha-C(3a)); 2,545 (dd,J=19,J=6,8,Ha-C(3)); 2,29-2,22 (m,Ha-C(4); 1,95 (dd,J=19,J=2,Hβ-C(3)); 1,18 (s,$CH_3$-C(9a)); 0,93 (s,$CH_3$-C(6)); 0,895 (s,$CH_3$-C(6));
$^{13}$C-NMR ($CDCl_3$): 194,5 (s,C(2)); 121,9 (d,C(1)); 53,67 (d,C(5a)); 42,4 (t,C(3)); 41,8 (t,C(7)); 39,9 (s,C(9a)); 38,3 (d,C(3a)); 37,0 (t,C(9)); 35,8 (t,C(4)); 33,9 (s,C(6)); 33,3 (q,C(11)); 21,7 (q,C(10)); 21,3 (t,C(5)); 19,3 (q,C(12)); 18,4 (t,C(8));
MS: 232 (57 $M^+$), 217(12), 190(30), 175(30), 161(12), 147(19), 135(19), 122(26), 109(100), 91(40), 79(26), 69(27), 55(46), 41(77), 28(19).
$[\alpha]_D^{20}$ = -191,8 (c = 1,23 $CHCl_3$).
Geruch von (-)VII: schwach holzig, camphrig.

Verbindung (-)VIII

IR (liq): 1705, 1670, 1620
UV: α max: 235, ε = 14935, $CH_2Cl_2$
H-NMR ($CDCl_3$ 400 $MH_3$): 5,83 (m,J=1,4,H-C(3)); 2,85 (ddd,J=14,6,J=1,4,J=4,8,H-C(4)); 2,48 (d(breit), J=6,H-C(9b)); 2,32 (dd(breit),J=7,J=12,5,H-C(4)); 2,23 (ddd,J=19,J=6,5,J=1,Hβ-C(1)); 2,15 (ddd,J=19,J=1, J=2,5,Ha-C(1)); 1,91(ddd,J=13,4,J=2,4,J=6,4,Heq-C(5)); 1,27 (dd,J=12,5,J=2,6,H-C(5a)); 0,95 (s,$H_3$C(11); 0,86 (s,$H_3$-C(10)); 0,67 (s,$H_3$-C(12))
$^{13}$C-NMR ($CDCl_3$): 208,2 (s,C(2)); 181,7 (s,C(3a)); 126,7 (d,C(3)); 54,7 (d,C(9b)); 52,5 (d,C(5a)); 41,4 (t,C(7)); 39,5 (t,C(9)); 38,3 (s,C(9a)); 35,4 (t,C(1)); 32,8 (q,C(11)); 32,5 (s,C(6)); 30,0 (t,C(4)); 21,9 (t,C(5)); 21,1 (q,C(10)); 18,0 (t,C(8)); 12,0 (q,C(12))
MS: $M^+$ 232(11); 217(9); 204(5); 199(1); 189(5,6); 175(4,4); 161(8,4); 149(10); 137(72); 123(38); 109(57); 96(100); 91(16); 81(40); 69(33,5); 55(29); 41(48)
$[\alpha]_D^{20}$ = -103,5° (c = 1,04 $CHCl_3$).
Geruch von VIII: schwach holzig.

Beispiel 2

Man löst zunächst 24 g Phosphorpentoxid in 240 g Methansulfonsäure, 50 g (+)-Sclareolid II' werden in 250 g dieser Lösung während 20 Minuten auf 106°C erhitzt. Man lässt noch 30 Minuten bei 104°C rühren. Dann wird mit einem Eisbad abgekühlt, mit Eiswasser versetzt und mit Aether extrahiert. Die organische Phase wird mit 2 NaOH, mit gesättigter Kochsalzlösung gewaschen, getrocknet und eingedampft. Das Rohprodukt (31,5 g) wird destilliert. Man erhält auf diese Weise zwei Fraktionen. Die nieder siedende Fraktion, Sdp. 115-126°C (0,045 mm) 9,5 g (20%) enthält (GC) 3% (-)I', 13% (-)I", 8,4% (-)X, 19% (-)VII und 50% (-)VIII. Die höher siedende Fraktion 119-126°C (0,045) 11,5 g (25%) enthält (GC) 26% (-)VII und 70% (-)VIII.

Die Verbindung X kann durch Chromatographie an Kieselgel 60 (0,04-0,063 mm) gereinigt werden.

Spektrale Daten von X:

IR (KBr): 1695, 1640 $cm^{-1}$
UV (EtOH) λ max: 242,4, ε = 13025 (Reinheit 75%)
H-NMR (400 $MH_3$) $CDCl_3$: 2,51 (d,J=18,5,Hβ-C(2)); 1,99 (d,J=18,5,Ha-C(2)); 1,74 (m,Heq-C(5)); 1,32 (s,$H_3$-C(9)); 0,95 (s,$H_3$-C(12)); 0,87 (s,$H_3$-C(11)); 0,78 (s,d,J=7, $H_3$-C(10))
$^{13}C$ ($CDCl_3$): 206,7 (s,C(1)); 179,4 (s,C(8b)); 134,8 (s,C(8a)); 47,4 (t,C(2)); 44,3 (s,C(2a)); 42,9 (d,C(5a)); 37,4 (d,C(3)); 33,8 (t,C(7)); 31,5 (s,C(6)); 28,0 (t,C(4)); 27,6 (q,C(12)); 26,5 (q,C(9)); 23,8 (t,C(11)); 22,0 (t,C(5)); 16,7 (t,C(8)); 14,45 (q,C(10)).
MS: 232(100); 217(28); 203(4); 180(28); 176(55); 161(45); 148(43); 134(47); 119(31); 105(38); 91(50); 77(22); 69(15); 65(11); 55(20); 41(37); 29(8).

Beispiel 3

15 g Polyphosphorsäure werden auf 100°C erwärmt. 4 g (-)-Isosclareolid II''' (Decahydro-3a,6,6,9a-tetramethyl-[3aS(3aβ,5aβ,9aa,9bβ)]naphtho[2,1-b]furan-2(1H)-on) werden zugegeben und 15 Minuten bei dieser Temperatur gerührt. Dann wird auf Wasser gegossen, mit Methylenchlorid extrahiert, neutral gewaschen und eingedampft. Das Rohprodukt (3 g) wird über 150 g Kieselgel mit 20% und 50% Hexan/Aether eluiert. Man erhält auf diese Weise 0,74 g (20%) eines Gemisches von (-)I'/I'' im Verhältnis 16/84 und 0,384 g (10%) (-)VII und (-)VIII im Verhältnis 5/92.

Beispiel 4

11,3 g Polyphosphorsäure werden vorgelegt und auf 100°C erwärmt. 3 g (-)Episclareolid II'' (Decahydro-3a,6,6,9a-tetramethyl-[3aR(3aα,5aβ,9aa,9ba]naphtho[2,1-b]furan-2(1H)-on) werden zugegeben und 15 Minuten bei dieser Temperatur gerührt. Dann wird auf Wasser gegossen, mit Methylenchlorid extrahiert, neutral gewaschen und eingedampft. Das Rohprodukt (1,9 g) wird über 100 g Kieselgel mit 20% und 50% Hexan/Aether eluiert. Man erhält auf diese Weise 359 mg (12,9%) I' und I'' im Verhältnis 43/56 und 180 mg (6%) VII und VIII im Verhältnis 9/87.

Beispiel 5

Zu 1 g eines Gemisches von (-)I'/I'' im Verhältnis 1/3, gelöst in 10 ml Aether werden 150 mg Lithiumaluminiumhydrid gegeben. Dann wird 20 Stunden bei Raumtemperatur gerührt. Der Ueberschuss Lithiumaluminiumhydrid wird mit wenig Aethylacetat zerstört, Wasser und 2N HCl zugegeben, ausgeschüttelt und eingedampft. Auf diese Weise erhält man 1 g Rückstand. Dieser wird über 90 g Kieselgel 60 (230-400 mesh) mit 10% und 20% Aether in Hexan eluiert. Auf diese Weise erhält man 225 mg Kohlenwasserstoff XI und 150 mg (-)I'''. Der Kohlenwasserstoff (-)XI wird durch eine nochmalige Chromatographie über Kieselgel 60 (0,04-0,063) 10% $AgNO_3$ chromatographiert. Auf diese Weise erhält man 70 mg reines (-)XI.

Spektrale Daten von (-)XI:

IR (flüssig): 1635, 870, 810 $cm^{-1}$
UV (EtOH); λ max: 236, ε = 13711
$^1$H-NMR (400 $MH_3$, $CDCl_3$): 5,97 (d,J=5,5,H-C(7) oder H-C(8)); 5,915 (d,J=5,5,H-C(7) oder H-C(8)); 4,837 (s,H-C(15)); 4,607 (s,H-C(15)); 0,938 (s,$CH_3$-(C5)), 0,807 (d,J=6,5,$CH_3$-C(10)); 0,707 (s,$CH_3$-(C5));
$^{13}$C-NMR ($CDCl_3$): 160,4 (s); 145,6 (d); 130,2 (d); 101,04 (t); 65,6 (s); 56,3 (s); 45,8 (d); 35,2 (s); 34,1 (t); 33,4 (t); 31 (t); 27,9 (q), 26,9 (q); 22,5 (t); 17,7 (q), 13,1 (q)
MS: 216(23); 201(13); 188(1,6); 173(26); 159(14); 145(23); 134(100); 119(64); 105(35); 91(39); 77(11); 65(9); 55(19); 41(30); 32(10); 28(64)
$[\alpha]_D$ ($CHCl_3$): -102,5°, c = 1,034.

Beispiel 6

16,6 g (0,071M) eines Gemisches von (-)I' und (-)I'' im Verhältnis 1/3,3, gelöst in 140 ml Toluol werden bei Raumtemperatur vorgelegt und innerhalb von 37 Minuten mit 72 ml (0,251M) Natrium-bis-(2-methoxyäthoxy)aluminiumdihydrid (70% in Toluol; "Fluka") zugetropft. Das Reaktionsgemisch wird auf 78°C erhitzt und während 23 Stunden und 15 Minuten gerührt. Es wird auf 6°C abgekühlt und das Reaktionsgemisch auf Eiswasser gegossen. Es werden 2N NaOH und Aether zugegeben und während 1 Stunde gerührt. Die wässrige Phase wird mit 2N HCl und gesättigter Kochsalzlösung gewaschen, getrocknet und eingedampft. Das Rohprodukt (16,96 g) wird bei 6,7 Pa (0,05 Torr) destilliert. Auf diese Weise erhält man 8,65 g (51,8%) Produkt vom Sdp. 92-96°C. Dieses Produkt enthält (GC) 65% (-)I''', 20% (-)I' und 7% (-)I''. Die Verbindung I''' kann nachträglich mittels Chromatographie (Kieselgel 60, 0,04-0,063 mm) und Elution mit 5% Aether/Hexan gereinigt werden.

Spektrale Daten von (-)I''':

IR (liq) 1690, 1730 $cm^{-1}$
$^{1}H$-NMR (400 $MH_3$, $CDCl_3$): 2,56 (m,J=9,6,J=9,6, J=6,8,H-C(9)); 2,43 (dd,J=20,J=9,6,H-C(8) anti zu $CH_3$-C(9)); 2,04 (dd,J=20,J=9,6,H-C(8) syn zu $CH_3$-C(9)); 1,06 (d,J=6,8,$H_3$-C(15)); 0,95 (s,$H_3$-C(14)); 0,92 (d,J=6,5,$H_3$-C(16)); 0,85 (s,$H_3$-C(13));
$^{13}C$-NMR ($CDCl_3$): 221,2 (s,C(7)); 66,6 (s,C(6)); 52,0 (s,C(1)); 43,8 (t,C(8)); 38,3 (d,C(10)); 36,1 (t,C(4)); 32,7 (s,C(5)); 31,6 (d,C(9)); 30,1 (t,C(11)); 27,4 (t,C(12)); 27,2 (q,C(13)); 24,5 (q,C(14)); 22,8 (t,C(2)); 17,2 (t,C(3)); 15,7 (q,C(16)); 15,4 (q,C(15))
$[\alpha]_D^{20}$ = -27,56 (c = 1,10, $CHCl_3$)
MS: 234(42); 221(21); 203(3); 191(8); 179(100); 163(35); 151(69); 149(88); 133(51); 121(35); 109(49); 93(56); 81(63); 69(69); 55(39); 43(56).
Geruch von (-)I''': graue Ambra, Ambroxan

<u>Beispiel 7</u>

Aus β,γ-Monocyclo-homofarnesylsäure (Z/E: 1/2) [G. Lucius, Angew. Chem. (1956) <u>7</u>, 247] erhält man durch Behandlung mit Zinntetrachlorid in Toluol [A. Saito, H. Matsushita, H. Kaneko, Chemistry Letters (1983), 729] bei -20°C oder Trifluoressigsäure bei 0°C [EP-A 0 165 458] ein Gemisch von (±)-Episclareolid II'' und (±)-Sclareolid II' im Verhältnis 4/3. Behandelt man dieses racemische Gemisch mit Polyphosphorsäure bei 100°C, erhält man die Propellanderivate (±)I' und (±)I'' im Verhältnis 1/3 mit 34% Ausbeute und die Cyclopentenone (±)VII und (±)VIII im Verhältnis 1/4 mit 13% Ausbeute.

<u>Beispiel 8</u>

150 g Polyphosphorsäure wird vorgelegt und auf 100°C erhitzt. 40 g eines Gemisches von (±)-Episclareolid II'' und (±)-Sclareolid II' (im Verhältnis 4/3) werden bei 100°C geschmolzen und unter gutem Rühren zugegeben. Man lässt 1 Stunde bei 100-110°C unter intensivem Rühren reagieren, dann wird auf 70°C abgekühlt und mit Wasser und Hexan versetzt. Es wird unter Rühren auf Raumtemperatur abgekühlt, die Phasen werden getrennt, und die wässrige Phase wird mit Aether extrahiert. Die vereinigten organischen Phasen werden mit gesättigter Kochsalzlösung und mit gesättigter Bicarbonatlösung gewaschen, getrocknet und eingedampft. Auf diese Weise erhält man 29,1 g Rohprodukt. Eine Destillation ergibt 12,6 g (34%) eines Gemisches von (±)I' und (±)I'' im Verhältnis 1/2,5 (Sdp. 65-70°C 1,5 x $10^{-5}$ Torr) und 4,9 g (13%) eines Gemisches von (±)VII und (±)VIII im Verhältnis 1/4 (Sdp. 90-95°C 2 . $10^{-3}$ Pa (1,5 x $10^{-5}$ Torr)).

<u>Beispiel 9</u>

1S*,6R*,9S*,10S*-5,5,9,10-Tetramethyl-tricyclo-[4,3,3,$0^{1,6}$] dodecan-7-on (±)I''':

37 g (0,16M) eines Gemisches von (±)I' und (±)I'' im Verhältnis 1/2,5, gelöst in 140 ml Toluol, wird bei Raumtemperatur vorgelegt und innerhalb 45 Minuten werden 137 ml (0,479M) Natrium-bis-(2-methoxyäthoxy)aluminiumdihydrid (70% in Toluol) zugetropft. Das Reaktionsgemisch wird anschliessend auf 78°C erhitzt und während 41 Stunden gerührt. Es wird auf 6°C abgekühlt. Das Reaktionsgemisch wird auf Eiswasser gegossen. Es wird NaOH 30% und Aether zugegeben und während 1 Stunde gerührt. Die wässrige Phase wird mit Toluol extrahiert, die organische Phase mit 2N HCl und gesättigter Kochsalzlösung gewaschen, getrocknet und eingedampft. Das Rohprodukt (37,4 g) wird bei 2 . $10^{-3}$ Pa (1,5 x $10^{-5}$ Torr) destilliert. Auf diese Weise erhält man 17 g (45%) Produkt, Sdp. 75°C. Dieses Produkt enthält (GC) 61% (±)I''', 25% (±)I' und 6% (±)I''.

In den folgenden Beispielen steht A für das
Gemisch mit
65% (-)I'''
20% (-)I'

7% (-)I"

und B für das Gemisch mit

61% (±)I'"

25% (±)I'

6% (±)I"

Beispiel 10

Komposition für Eau de toilette:

| | Gewichtsteile | |
|---|---|---|
| A oder B | | 70,00 |
| Benzylacetat | 40,00 | 40,00 |
| Geranylacetat | 50,00 | 50,00 |
| Allylamylglycolat | 2,00 | 2,00 |
| Methylanthranilat | 1,00 | 1,00 |
| Basilikum-Essenz | 10,00 | 10,00 |
| Bergamotte-Essenz | 250,00 | 250,00 |
| Carbitol | 170,00 | 100,00 |
| Ceton alpha (Givaudan) | 50,00 | 50,00 |
| Citron-Essenz (Argentin.) | 150,00 | 150,00 |
| Coumarin crist. | 20,00 | 20,00 |
| Estragon-Essenz | 5,00 | 5,00 |
| Fixolid (Givaudan) | 40,00 | 40,00 |
| Nelkenknospenessenz | 15,00 | 15,00 |
| Hedion (Firmenich) | 50,00 | 50,00 |
| Isoeugenol | 3,00 | 3,00 |
| Lavandin Ess. | 100,00 | 100,00 |
| Evernyl (Roure Bertrand) | 2,00 | 2,00 |
| Muskatnussessenz | 20,00 | 20,00 |
| Pêche pure (Givaudan) | 2,00 | 2,00 |
| Sandalore (Givaudan) | 20,00 | 20,00 |
| | 1000,00 | 1000,00 |

Beispiel 11

Formulierung für Kosmetika:

| | Gewichtsteile | |
|---|---|---|
| A oder B | | 30,00 |
| Benzylacetat | 100,00 | 100,00 |
| Geranylacetat | 100,00 | 100,00 |
| Butylcyclohexylacetat | 100,00 | 100,00 |
| Verdylacetat (Givaudan) | 20,00 | 20,00 |
| Phenyläthylalkohol | 150,00 | 150,00 |
| Hexylzimtaldehyd | 100,00 | 100,00 |
| Bergamotte Ess. (Calabrien) | 200,00 | 200,00 |
| Cyclohexylallylpropionat | 1,00 | 1,00 |
| Dimetol (Givaudan) | 20,00 | 20,00 |
| Dipropylenglykol | 30,00 | |
| Gardenol (Givaudan) | 2,00 | 2,00 |
| Isoeugenol | 2,00 | 2,00 |
| Linalool synt. | 50,00 | 50,00 |
| Geraniumoxid (Givaudan) 10%/DIP | 5,00 | 5,00 |
| Petitgrain Ess. Paraguay | 20,00 | 20,00 |
| Benzylsalicylat | 100,00 | 100,00 |
| | 1000,00 | 1000,00 |

Die Zugabe von A bzw. B im Beispiel 10 und Beispiel 11, die sich durch eine trockene Holznote (Zedernholz) mit Weihrauch- und Ambra-Einschlag auszeichnen, verschafft der Komposition Fülle und Reichtum. Die würzige Note der Komposition wird insbesondere schön abgerundet.

## Patentansprüche

### Patentansprüche für folgende Vertragsstaaten : CH, DE, FR, GB, IT, LI, NL

1. Verbindungen der Formel

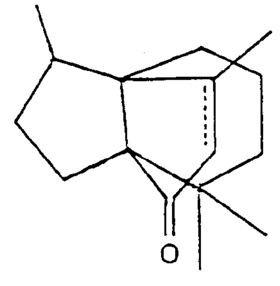

I

worin die gestrichelte Linie eine fakultative Bindung bedeutet, als Racemate oder einzelne optische Antipoden.

2. (±)1S*,6R*,10S*-5,5,9,10-Tetramethyl-tricyclo-[4,3,3,$0^{1,6}$]dodec-8-en-7-on.

3. (±)1S*,6R*,10R*-5,5,9,10-Tetramethyl-tricyclo-[4,3,3,$0^{1,6}$] dodec-8-en-7-on.

4. (±)1S*,6R*,9S*,10S*-5,5,9,10-Tetramethyl-tricyclo-[4,3,3,$0^{1,6}$]dodecan-7-on.

5. (-)-1S,6R,10S-5,5,9,10-Tetramethyl-tricyclo-[4,3,3,$0^{1,6}$]dodec-8-en-7-on.

6. (-)1S,6R,10R-5,5,9,10-Tetramethyl-tricyclo-[4,3,3,$0^{1,6}$]dodec-8-en-7-on.

7. (-)1S,6R,9S,10S-5,5,9,10-Tetramethyl-tricyclo-[4,3,3,$0^{1,6}$]dodecan-7-on.

8. Riechstoffkomposition, gekennzeichnet durch einen Gehalt an einer Verbindung der Formel

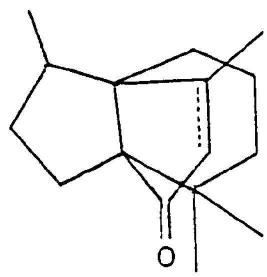

I

worin die gestrichelte Linie eine fakultative Bindung bedeutet.

9. Verfahren zur Herstellung der Verbindungen der Formel I gemäss Anspruch 1, dadurch gekennzeichnet, dass man Sclareolid, Episclareolid oder Isosclareolid bei erhöhter Temperatur in saurem Medium unter Wasserabspaltung umlagert, und die im Reaktionsprodukt vorhandene C-C-Doppelbindung gegebenenfalls reduziert.

10. Verfahren nach Anspruch 9, dadurch gekennzeichnet, dass man Sclareolid mit einem phosphorhaltigen Umlagerungsmittel, insbesondere mit Polyphosphorsäure oder mit $P_2O_5$/Methansulfonsäure umsetzt.

11. Verfahren nach Anspruch 9, dadurch gekennzeichnet, dass man mit Natrium-bis-(2-methoxyäthoxy)aluminiumdihydrid (Redal) reduziert.

12. Verwendung einer Verbindung der Formel I gemäss Anspruch 1 als Riechstoff.

**Patentansprüche für folgenden Vertragsstaat : ES**

1. Verfahren zur Herstellung der Verbindungen der Formel

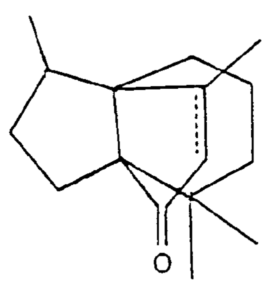

I

worin die gestrichelte Linie eine fakultative Bindung bedeutet, als Racemate oder einzelne optische Antipoden, dadurch gekennzeichnet, dass man Sclareolid, Episclareolid oder Isosclareolid bei erhöhter Temperatur in saurem Medium unter Wasserabspaltung umlagert, und die im Reaktionsprodukt vorhandene C-C-Doppelbindung gegebenenfalls reduziert.

2. Verfahren nach Anspruch 1, dadurch gekennzeichnet, dass man Sclareolid mit einem phosphorhaltigen

Umlagerungsmittel, insbesondere mit Polyphosphorsäure oder mit $P_2O_5$/Methansulfonsäure umsetzt.

3. Verfahren nach Anspruch 1, dadurch gekennzeichnet, dass man mit Natrium-bis-(2-methoxyäthoxy)aluminiumdihydrid (Redal) reduziert.

4. Verfahren nach einem der Ansprüche 1 bis 3, dadurch gekennzeichnet, dass man
   (±)1S*,6R*,10S*-5,5,9,10-Tetramethyl-tricyclo-[4,3,3,$0^{1,6}$]dodec-8-en-7-on,
   (±)1S*,6R*,10R*-5,5,9,10-Tetramethyl-tricyclo-[4,3,3,$0^{1,6}$]dodec-8-en-7-on,
   (±)1S*,6R*,9S*,10S*-5,5,9,10-Tetramethyl-tricyclo-[4,3,3,$0^{1,6}$]dodecan-7-on,
   (-)-1S,6R,10S-5,5,9,10-Tetramethyl-tricyclo-[4,3,3,$0^{1,6}$]dodec-8-en-7-on,
   (-)1S,6R,10R-5,5,9,10-Tetramethyl-tricyclo-[4,3,3,$0^{1,6}$]dodec-8-en-7-on,
   (-)1S,6R,9S,10S-5,5,9,10-Tetramethyl-tricyclo-[4,3,3,$0^{1,6}$]dodecan-7-on
   herstellt.

5. Riechstoffkomposition, gekennzeichnet durch einen Gehalt an einer Verbindung der Formel

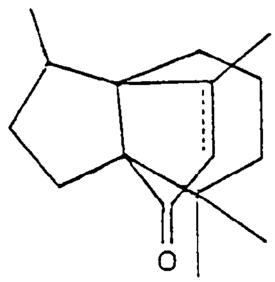

I

worin die gestrichelte Linie eine fakultative Bindung bedeutet.

6. Verwendung einer Verbindung der Formel I gemäss Anspruch 1 als Riechstoff.

## Claims

### Claims for the following Contracting States : CH, DE, FR, GB, IT, LI, NL

1. Compounds of the formula

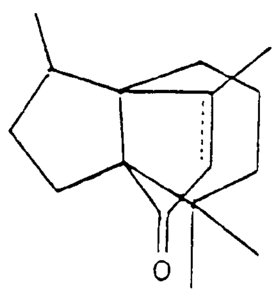

I

wherein the dotted line signifies an optional bond, as racemates or individual optical antipodes.

2. (±)1S*,6R*,10S*-5,5,9,10-Tetramethyl-tricyclo-[4,3,3,$0^{1,6}$]dodec-8-en-7-one.

3. (±)1S*,6R*,10R*-5,5,9,10-Tetramethyl-tricyclo-[4,3,3,$0^{1,6}$]dodec-8-en-7-one.

4. (±)1S*,6R*,9S*,10S*-5,5,9,10-Tetramethyl-tricyclo-[4,3,3,$0^{1,6}$]dodecan-7-one.

5. (-)-1S,6R,10S-5,5,9,10-Tetramethyl-tricyclo-[4,3,3,$0^{1,6}$]dodec-8-en-7-one.

6. (-)1S,6R,10R-5, 5,9,10-Tetramethyl-tricyclo-[4,3,3,$0^{1,6}$]dodec-8-en-7-one.

7. (-)1S,6R,9S,10S-5,5,9,10-Tetramethyl-tricyclo-[4,3,3,$0^{1,6}$]dodecan-7-one.

8. An odorant composition, characterized in that it contains a compound of the formula

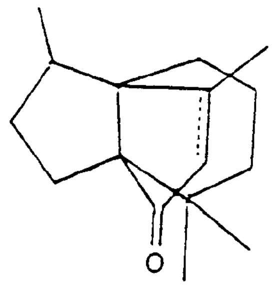

I

wherein the dotted line signifies an optional bond.

9. A process for the manufacture of the compounds of formula I in accordance with claim 1, characterized by rearranging sclareolid, episclareolid or isosclareolid at an elevated temperature in acidic medium with the cleavage of water and, if desired. reducing the C-C double bond present in the reaction product.

10. A process according to claim 9, characterized in that sclareolid is reacted with a phosphorus-containing rearrangement agent, especially with polyphosphoric acid or with $P_2O_5$/methanesulphonic acid.

11. A process according to claim 9, characterized in that the reduction is carried out with sodium bis-(2-methoxyethoxy)aluminium hydride (Redal).

12. The use of a compound of formula I in accordance with claim 1 as an odorant.

**Claims for the following Contracting State : ES**

1. A process for the manufacture of compounds of the formula

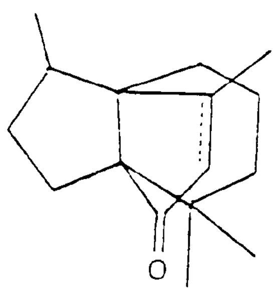

I

wherein the dotted line signifies an optional bond, as racemates or individual optical antipodes, characterized by rearranging sclareolid, episclareolid or isosclareolid at an elevated temperature in acidic medium with the cleavage of water and, if desired, reducing the C-C double bond present in the reaction product.

2. A process according to claim 1, characterized in that sclareolid is reacted with a phosphorus-containing rearrangement agent, especially with polyphosphoric acid or with $P_2O_5$/methanesulphonic acid.

3. A process according to claim 1, characterized in that the reduction is carried out with sodium bis-(2-methoxyethoxy)aluminium hydride (Redal).

4. A process according to any of claims 1 to 3, characterized in that
(±)1S*,6R*,10S*-5,5,9,10-tetramethyl-tricyclo-[4,3,3,$0^{1,6}$]dodec-8-en-7-one,

(±)1S*,6R*,10R*-5,5,9,10-tetramethyl-tricyclo-[4,3,3,$0^{1,6}$]dodec-8-en-7-one,
(±)1S*,6R*,9S*,10S*-5,5,9,10-tetramethyl-tricyclo-[4,3,3,$0^{1,6}$]dodecan-7-one,
(-)-1S,6R,10S-5,5,9,10-tetramethyl-tricyclo-[4,3,3,$0^{1,6}$]dodec-8-en-7-one,
(-)1S,6R,10R-5,5,9,10-Tetramethyl-tricyclo-[4,3,3,$0^{1,6}$]dodec-8-en-7-one,
(-)1S,6R,9S,10S-5,5,9,10-Tetramethyl-tricyclo-[4,3,3,$0^{1,6}$]dodecan-7-one,
is manufactured.

5. An odorant composition, characterized in that it contains a compound of the formula

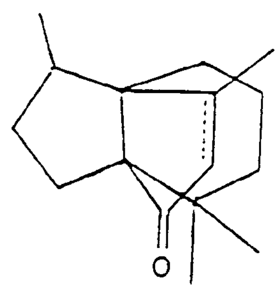

I

wherein the dotted line signifies an optional bond.

6. The use of a compound of formula I in accordance with claim 1 as an odorant.

**Revendications**

**Revendications pour les Etats contractants suivants : CH, DE, FR, GB, IT, LI, NL**

1. Composés de formule

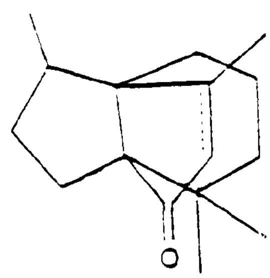

I

dans laquelle le trait interrompu indique une liaison facultative, à l'état de racémates ou d'antipodes optiques isolés.

2. La (±)1S*,6R*,10S*-5,5,9,10-tétraméthyl-tricyclo-[4,3,3,$0^{1,6}$]dodéca-8-ène-7-one.

3. La (±)1S*,6R*,10R*-5,5,9,10-tétraméthyl-tricyclo-[4,3,3,$0^{1,6}$]dodéca-8-ène-7-one.

4. La (±)1S*,6R*,9S*,10S*-5,5,9,10-tétraméhtyl-tricyclo-[4,3,3,$0^{1,6}$]dodécane-7-one.

5. La (-)-1S,6R,10S-5,5,9,10-tétraméthyl-tricyclo-[4,3,3,$0^{1,6}$]dodéca-8-ène-7-one.

6. La (-)1S,6R,10R-5,5,9,10-tétraméthyl-tricyclo-[4,3,3,$0^{1,6}$]dodéca-8-ène-7-one.

7. La (-)1S,6R,9S,10S-5,5,9,10-tétraméthyl-tricyclo-[4,3,3,$0^{1,6}$]dodécane-7-one.

8. Composition de parfum caractérisée en ce qu'elle contient un composé de formule

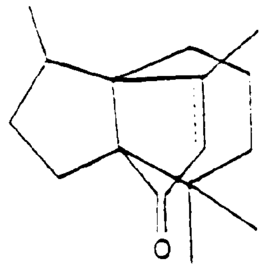

I

dans laquelle le trait interrompu indique une liaison facultative.

**9.** Procédé de préparation des composés de formule I selon la revendication 1, caractérisé en ce que l'on soumet le sclaréolide, l'épisclaréolide ou l'isosclaréolide à transposition à chaud, en milieu acide, avec séparation d'eau, et le cas échéant on réduit la double liaison C-C existant dans le produit de réaction.

**10.** Procédé selon la revendication 9, caractérisé en ce que l'on fait réagir le sclaréolide avec un agent de transposition phosphoré, en particulier l'acide poly-phosphorique, ou avec le couple $P_2O_5$/acide méthane-sulfonique.

**11.** Procédé selon la revendication 9, caractérisé en ce que l'on réduit à l'aide du dihydrure de sodium et de bis-(2-méthoxyéthoxy)-aluminium (Redal).

**12.** Utilisation d'un composé de formule I selon la revendication 1 en tant que matière odorante.

**Revendications pour l'Etat contractant suivant : ES**

**1.** Procédé de préparation des composés de formule

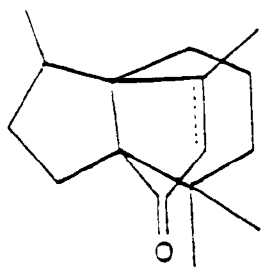

I

dans laquelle le trait interrompu indique une liaison facultative, à l'état de racémates ou d'antipodes optiques isolés, caractérisé en ce que l'on soumet le sclaréolide, l'épisclaréolide ou l'isosclaréolide à transposition à chaud, en milieu acide, avec séparation d'eau et le cas échéant on réduit la double liaison C-C existant dans le produit de réaction.

**2.** Procédé selon la revendication 1, caractérisé
en ce que l'on fait réagir le sclaréolide avec un agent de transposition phosphoré, en particulier l'acide polyphosphorique, ou avec le couple $P_2O_5$/acide méthane-sulfonique.

**3.** Procédé selon la revendication 1, caractérisé en ce que l'on réduit à l'aide du dihydrure de sodium et de bis-(2-méthoxyéthoxy)-aluminium (Redal).

**4.** Procédé selon la revendication 1 à 3, caractérisé en ce que l'on prépare :

La (±)1S*,6R*,10S*-5,5,9,10 tétraméthyl-tricyclo-[4,3,3,$0^{1,6}$]dodéca-8-ène-7-one.
La (±)1S*,6R*,10S*-5,5,9,10-tétraméthyl-tricyclo-[4,3,3,$0^{1,6}$]dodéca-8-éne-7-one.
La (±)1S*,6R*,9S*,10S*5,5,9,10-tétraméthyl-tricyclo-[4,3,3,$0^{1,6}$]dodécane-7-one.
La (-)-1S,6R,10S-5,5,9,10-tétraméthyl-tricyclo-[4,3,3,$0^{1,6}$]dodéca-8-ène-7-one.
La (-)1S,6R,10R-5,5,9,10-tétraméthyl-tricyclo-[4,3,3,$0^{1,6}$]dodéca-8-ène-7-one.
La (-)1S,6R,9S,10S-5,5,9,10-tétraméthyl-tricyclo-[4,3,3,$0^{1,6}$]dodécane-7-one.

5. Composition de parfum, caractérisée en ce qu'elle contient un composé de formule

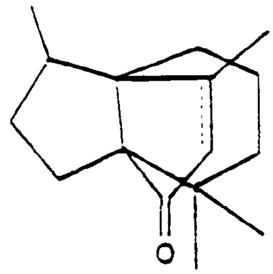

I

dans laquelle le trait interrompu indique une liaison facultative.

6. Utilisation d'un composé de formule I selon la revendication 1 en tant que matière odorante.